# EUROPÄISCHE PATENTSCHRIFT

(11) **EP 1 979 463 B1**
(45) Veröffentlichungstag und Bekanntmachung des Hinweises auf die Patenterteilung: **30.12.2009**
(21) Anmeldenummer: 06818612.1
(22) Anmeldetag: 17.11.2006
(51) Int. Cl.: C12M 1/00

(54) **VORRICHTUNG UND VERFAHREN ZUM INKUBIEREN VON ZELLEN**
DEVICE AND METHOD FOR THE INCUBATION OF CELLS
DISPOSITIF ET PROCÉDÉ D'INCUBATION DE CELLULES

(30) Priorität: 30.01.2006 DE 102006004157
(43) Veröffentlichungstag der Anmeldung: 15.10.2008
(73) Patentinhaber: EPPENDORF AG, 22339 Hamburg (DE)
(72) Erfinder: BARTSCH, Olaf, 22523 Hamburg (DE)
(74) Vertreter: Hauck Patent- und Rechtsanwälte
(86) Internationale Anmeldenummer: PCT/EP2006/011032
(87) Internationale Veröffentlichungsnummer: WO 2007/085284

(56) Entgegenhaltungen:
- WO-A-03/069351
- DE-A1- 10 244 859
- DE-A1- 19 646 115
- DE-U1- 29 917 313
- FR-A1- 2 605 016
- US-A- 5 908 776
- US-B1- 6 593 136

## Beschreibung

Die Erfindung bezieht sich auf eine Vorrichtung und auf ein Verfahren zum Inkubieren von Zellen.

Herkömmlicherweise werden Zellen zum Inkubieren in ein Kulturgefäß eingefüllt und wird das Kulturgefäß in einen Brutschrank eingesetzt. Im Brutschrank werden optimale Bedingungen für die Inkubation der Zellkulturen geschaffen, indem eine hierfür geeignete Atmosphäre und Temperatur (z.B. 37°C) eingestellt wird. Die Atmosphäre wird in der Regel durch Luft mit einem bestimmten CO2- und 02-Gehalt und einer bestimmten Luftfeuchtigkeit gebildet. Das Kulturgefäß ist beispielsweise eine mehrere Aufnahmen aufweisende Multiwell-Kulturplatte, eine Kulturflasche oder eine Kulturschale. Die Zellen werden durch loses Auflegen eines Deckels auf Kulturplatten und Kulturschalen geschützt. Der lose aufliegende Deckel ermöglicht es, daß die im Brutschrank herrschende Atmosphäre an den Zellen auf der Kulturplatte bzw. der Kulturschale anliegt. Kulturflaschen haben einen Verschluß mit einem integrierten Filter, der ebenfalls gewährleistet, daß die Atmosphäre an den Zellen anliegt.

Im Zellkulturlabor teilen sich in der Regel mehrere Anwender einen großen Brutschrank. Die Kulturbedingungen im Brutschrank können durch verschiedene Anwender beeinflußt werden. So ist nacht ausgeschlossen, daß verschiedene Anwender Kulturbedingungen verändern, so daß sie für die Inkubation ihrer speziellen Zellkulturen optimal sind. Ferner besteht die Gefahr von Kreuz-Kontaminationen, z.B. mit Mycoplasmen. Durch das mehrmalige Öffnen der Türen und Einbringen von neuem Zellmaterial können die Anwender neue Kontaminationen einführen. Ferner können sie die Kulturgefäße anderer Anwender an einen anderen Platz stellen und dadurch das Wiederauffinden erschweren bzw. Verwechslungen verursachen.

Davon ausgehend liegt der Erfindung die Aufgabe zugrunde, eine Vorrichtung und ein Verfahren zum Inkubieren von Zellen zu schaffen, die bzw. das die Einhaltung der gewünschten Kulturbedingungen bei der Inkubation von Zellkulturen fördert.

Die Aufgabe wird durch eine Vorrichtung mit den Merkmalen des Anspruches 1 gelöst. Ferner wird sie durch ein Verfahren mit den Merkmalen des Anspruches 27 gelöst. Vorteilhafte Ausgestaltungen der Vorrichtung und des Verfahrens sind in den Unteransprüchen angegeben.

Die erfindungsgemäße Vorrichtung zum Inkubieren von Zellen hat einen sterilen oder sterilisierbaren, tragbaren Behälter zum kontaminationsdichten Umschließen mindestens eines integrierten und/oder einsetzbaren Kulturgefäßes zur Aufnahme von Zellen mit mindestens einer verschließbaren Öffnung zum Einbringen und/oder Entnehmen von Zellen und/oder Kulturmedium und/oder eines Kulturgefäßes in den und/oder aus dem Behälter und mindestens eine Einrichtung zum Herstellen von Kulturbedingungen in dem Behälter, die mindestens einen Sterilfilter in mindestens einer Wandung des Behälters umfasst.

Bei dem erfindungsgemäßen Verfahren zum Inkubieren von Zellen unter Verwendung einer erfindungsgemäßen Vorrichtung wird mindestens ein Zellen enthaltendes Kulturgefäß von einem sterilen, tragbaren Behälter kontaminationsdicht umschlossen bereitgestellt und werden im Behälter Kulturbedingungen hergestellt, wobei ein Kulturbedingungen aufweisendes Gas sterilgefiltert und in den Behälter eingeleitet wird.

Erfindungsgemäß werden die Kulturgefäße zum Inkubieren in einen Behälter verbracht bzw. sind in einem Behälter integriert. Unter einem Kulturgefäß wird eine Multiwell-Kulturplatte, Kulturflasche oder ein anderer für die Inkubation von Zellkulturen geeigneter Kulturträger verstanden. Wenn das Kulturgefäß in den Behälter integriert ist, bildet es nur einen Teil des Behälters. Eine Kulturflasche, die zugleich ein Kulturgefäß und ein kontaminationsdicht geschlossener Behälter ist, ist nicht Bestandteil der Erfindung. Der Behälter schließt die spezifische Zellkultur steril gegen die Umgebung ab. In dem Behälter werden die Kulturbedingungen hergestellt, wie z.B. Temperatur, Atmosphäre (z.B. CO2- und 02- und Wasserdampfgehalt). Die Kulturbedingungen werden beispielsweise in einem Brutschrank hergestellt und in den Behälter eingebracht. Gemäß einem weiteren Beispiel werden sie durch Begasung des Kulturgefäßes im Behälter mit einer definierten und gegebenenfalls temperierten Atmosphäre hergestellt. Die Begasung kann mittels einer geeigneten Begasungseinrichtung vorgenommen werden. Gemäß einem weiteren Beispiel wird durch Wärmeübertragung zwischen einer Temperiereinrichtung und dem Behälter eine bestimmte Temperatur im Behälter hergestellt. Der Behälter ist z.B. ein wiederverwendbarer Behälter, der gegebenenfalls sterilisiert und wiederverwendet werden kann. Gemäß einem anderen Beispiel ist der Behälter ein Einmalteil, daß nach einmaligem Gebrauch weggeworfen werden kann.

Die Erfindung ermöglicht somit eine gegen unerwünschte Störung abgeschirmte, individuelle Zellkultur. Hierdurch können empfindliche Proben getrennt voneinander und ungestört inkubiert werden. Das Risiko der Kreuz-Kontamination, aber auch die allgemeine Kontaminationsgefahr wird durch die isolierte Inkubation gesenkt. Die Erfindung bietet Vorteile für Anwender mit geringem Platzangebot. Brutschränke können effektiver genutzt werden. Brutschränke müssen in regelmäßigen Abständen sorgfältig gereinigt werden. Bei verschmutzten und schwer zu reinigenden Brutschränken vermeidet die Erfindung die Kontamination der individuellen Zellkultur. Der Anwender wird flexibler, weil seine Abhängigkeit von der Infrastruktur im Labor sinkt. Falls die verfügbaren Brutschränke nicht ausreichen, um gleichzeitig mehrere verschiedene Bedingungen anzulegen, wie z.B. Inkubation bei 37 °C, bei 30 °C, von Zellinien und Primärzellen getrennt, eröffnet die Erfindung die Möglichkeit, auch ohne Brutschrank eine Inkubation unter speziellen Kulturbedingungen in dem Behälter durchzuführen, die durch Begasung des Behälters erzeugt werden können. Außerdem ermöglicht die Vorrichtung auch die Inkubation von Zellen außerhalb des Zellkulturlabors in einer anderen Arbeitsumgebung. Durch die Tragbarkeit des Behälters ist gewährleistet, daß sie vom Anwender gut handhabbar ist und platzsparend im Brutschrank oder anderswo plazierbar ist.

Die Erfindung kann für nicht-biologische und biologische Anwendungen, z.B. für die Inkubation von Bakterien, Pilzen, Hefen, höheren eukariotischen Zellen und Geweben genutzt werden. Die Anwendung empfiehlt sich besonders, wenn das biologische Material entweder sehr kostbar oder sehr empfindlich ist, z.B. bei Primärzell- oder Stammzellkulturen.

Gemäß einer Ausgestaltung umfaßt die Vorrichtung mindestens ein Kulturgefäß. Dieses ist z.B. in den Behälter einsetzbar.

Nach einer weiteren Ausgestaltung hat die Vorrichtung mindestens ein in den Behälter integriertes Kulturgefäß. Der Behälter ist also von vornherein mit einem Kulturgefäß ausgestattet. Das Kulturgefäß kann lose in den Behälter enthalten sein. Gemäß einer Ausgestaltung ist das Kulturgefäß fest und untrennbar mit dem Behälter verbunden. Behälter und Kulturgefäß sind z.B. originär einteilig hergestellt oder getrennt hergestellt und fest untrennbar zusammengefüge.

Gemäß einer Ausgestaltung sind Einrichtungen zum lösbaren Verbinden mindestens eines Kulturgefäßes mit dem Behälter vorhanden. Hierzu weisen Behälter und Kulturgefäß z.B. eine Klemm-, Steck- oder Rastverbindung oder eine andere geeignete Einrichtung zum lösbaren Verbinden auf.

Gemäß einer Ausgestaltung ist der Behälter für das Umschließen und/oder Einbringen und/oder Entnehmen mindestens einer Kulturplatte und/oder mindestens einer Kulturflasche und/oder mindestens einer Kulturschale geeignet. Somit weist der Behälter einen Innenraum mit einer Gestaltung und Abmessungen auf, die ein Umschließen mindestens eines der genannten Kulturgefäße ermöglichen und/oder weist mindestens eine Öffnung auf, die so bemessen und gestaltet ist, daß sie ein Einbringen und/oder Entnehmen mindestens eines der vorgenannten Kulturgefäße zuläßt.

Die Einrichtung zum Herstellen von Kulturbedingungen im Behälter kann unterschiedlich ausgeführt sein.

Die Einrichtung zum Herstellen von Kulturbedingungen umfasst mindestens einen Sterilfilter in mindestens einer Wandung des Behälters. Das Sterilfilter ermöglicht die Verwendung konventioneller Brutschränke zur passiven Begasung des Behälters. Die im Brutschrank herrschende Atmosphäre kann durch das Sterilfilter des eingesetzten Behälters in den Behälter eindringen, ohne die Zellkultur zu kontaminieren. Es ist möglich, eine bestimmte Temperatur im Behälter dadurch einzustellen, daß ein Gas mit der bestimmten Temperatur durch das Sterilfilter in den Behälter eintritt.

Das Sterilfilter nimmt beispielsweise eine oder mehrere Wandungen des Behälters teilweise oder ganz ein. Z.B. ist nur ein Teil einer oder mehrerer Wandungen als Sterilfilter ausgeführt. Gemäß einem anderen Beispiel sind sämtliche Wandungen des Behälters komplett als Sterilfilter ausgeführt.

Das Sterilfilter ist z.B. eine Porenmembran, wie sie bei bekannten Filter-Schraubverschlüssen für Zellkultur-, Suspensionskultur- und Rollerflaschen zum Einsatz kommt. Z.B. weist eine erfindungsgemäß einsetzbare, kommerzialisierte Kapillarporenmembran eine aus PET/PTFE eine Porengröße von etwa 0,2 µm auf. Hierbei handelt es sich um die Bio-One-Membrane der Firma Greiner (www.gbo.com/biosience).

Eine Membran ist z.B. durch randseitiges Verschweißen dicht mit einer Wandung eines Behälters aus einem geeigneten Kunststoff verbindbar, der mit dem Kunststoff der Membran verschweißbar ist. Gemäß einem weiteren Beispiel ist die Membran randseitig mit dem Material der Wandung umspritzt. Gemäß einem anderen Beispiel ist die Membran randseitig dicht auf die Wandung des Behälters aufgespannt, z.B. mittels eines den Rand der Membran überdeckenden Rahmens, der über geeignete Befestigungsmittel (z.B. Schrauben oder Nieten) mit dem Behälter verbunden ist oder bei Ausführung aus einem geeigneten Kunststoff am Umfang mit dem Behälter verschweißt ist. Gegebenenfalls ist zwischen der Membran und der Wandung des Behälters eine zusätzliche Dichtung.aus einem elastischen Material vorhanden. Der Behälter ist z.B. ein starrer oder mehr oder weniger flexibler Behälter.

Gemäß einer anderen Ausführung ist das Sterilfilter in mindestens eine Wandung des Behälters integriert, d.h. einteilig mit der Wandung ausgeführt. Hierfür ist z.B. eine Wandung teilweise oder ganz aus einem porösen Kunststoff (z.B. ein Schaumstoff) ausgeführt. Gegebenenfalls besteht der gesamte Behälter aus dem porösen Kunststoff. Der Behälter ist z.B. im Ein- oder Mehrkomponenten-Spritzgießverfahren aus dem porösen Kunststoff und gegebenenfalls mindestens einem weiteren Kunststoff hergestellt. Der Behälter ist z.B. ein starrer oder ein mehr oder weniger flexibler Behälter.

Gemäß einer Ausgestaltung umfaßt die Einrichtung zum Herstellen von Kulturbedingungen einen Brutschrank mit integrierter Temperiereinrichtung und Begasungseinrichtung zur Aufnahme mindestens eines Behälters. Die Temperiereinrichtung kontrolliert beispielsweise die Temperatur des von der Begasungseinrichtung gelieferten, in den Brutschrank eingeleiteten Gases. Gemäß einem anderen Beispiel steuert die Temperiereinrichtung die Temperatur der Abstellflächen, auf denen die Behälter mit den enthaltenen Kulturgefäßen abgestellt werden.

Gemäß einer Ausgestaltung umfaßt die Einrichtung zum Herstellen von Kulturbedingungen mindestens einen Anschluß des Behälters mit einem eine Wandung des Behälters querenden Durchgang für den Anschluß des Behälters an mindestens eine Begasungseinrichtung. Der Anschluß ermöglicht eine aktive Begasung und gegebenenfalls Temperierung des Behälters mittels externer Begasungs- und gegebenenfalls Temperierungseinrichtungen. Die Temperierung erfolgt z.B. durch ein temperiertes Gas und/oder über eine beheizbare Abstellfläche einer Begasungs- und Temperiereinrichtung. Eine Begasungs- und ggfs. Temperiereinrichtung ist z.B. als Dockingstation bzw. Versorgungsstation ausgeführt, an die mindestens ein tragbarer Behälter anschließbar ist.

Gemäß einer Ausgestaltung umfaßt die Einrichtung zum Herstellen von Kulturbedingungen eine Begasungseinrichtung und mindestens einen Schlauch, über den die Begasungseinrichtung mit mindestens einem Anschluß verbunden oder verbindbar ist.

Gemäß einer Ausgestaltung umfaßt der Anschluß ein Ventil zum Öffnen und Schließen des Durchgangs. Wenn das Ventil geschlossen ist, ist die Vorrichtung zum Inkubieren in einem konventionellen Brutschrank verwendbar.

Gemäß einer Ausgestaltung umfaßt die Einrichtung zum Herstellen von Kulturbedingungen mindestens ein in den Behälter integriertes und/oder in den Behälter einbringbares offenes Gefäß zum Verdampfen einer in das Gefäß eingegebenen Flüssigkeit. In das Gefäß, das z.B. als Schale oder Wanne ausgeführt ist, ist eine Flüssigkeit einfüllbar, z.B. steriles Wasser, damit sich im Behälter eine vom Dampf der Flüssigkeit gesättigte Atmosphäre ausbildet, welche das Verdampfen von Kulturmedium verhindert.

Gemäß einer Ausgestaltung umfaßt die Einrichtung zum Herstellen von Kulturbedingungen eine Mischeinrichtung, auf der der Behälter angeordnet oder anordenbar ist, um den Behälter einer Mischbewegung zu unterziehen.

Gemäß einer Ausgestaltung ist mindestens eine Öffnung des Behälters wiederverschließbar. Die Wiederverschließbarkeit der Öffnung ermöglicht es, den Behälter nach Entnahme einer Probe bzw. nach Entnahme und Wiedereinsetzen des Kulturgefäßes erneut für die Inkubation von Zellkulturen einzusetzen.

Die wiederverschließbare Öffnung weist z.B. eine Kappe mit einem Schraubverschluß oder Bajonettverschluß oder eine schwenkbare Klappe und ggfs. ein Dichtungselement auf, das bei geschlossener Kappe bzw. Klappe zwischen dieser und dem Rand der Öffnung des Behälters wirksam ist. Der Schraubverschluß bzw. Bajonettverschluß weist Gewinde bzw. Bajonett-Verbindungselemente an der Kappe und an der Öffnung des Behälters auf. Das Dichtelement ist beispielsweise ein Dichtring oder eine Dichtlippe aus einem elastischen Kunststoff oder aus Gummi.

Das Dichtelement ist z.B. einteilig mit der Kappe, der Klappe oder dem Behälter ausgebildet oder angesetzt oder eingespritzt. Die Klappe ist z.B. durch Verrasten oder mittels eines Riegels im geschlossenen Zustand am Behälter fixierbar.

Der Behälter weist gemäß einer Ausgestaltung mehrere verschiedene Öffnungen auf, wobei verschiedene Öffnungen verschiedenen Zwecken dienen, z.B. dem Einbringen und/oder Entnehmen von Zellen und/oder von Kulturmedium und/oder von anderen Flüssigkeiten und/oder Hilfsmitteln.

Gemäß einer Ausgestaltung weist der Behälter mindestens ein optisches Fenster zum Beobachten der Zellen im Kulturgefäß auf. Das optische Fenster hat eine Oberflächenqualität, die es ermöglicht, die Zellen im Kulturgefäß unter dem Mikroskop zu betrachten. Dies ermöglicht dem Anwender eine Analyse der Zellkultur unter Verwendung eines Mikroskopes. Gemäß einer Ausgestaltung weist das optische Fenster einen Abstand vom Zellkulturgefäß auf, der so gering (z.B. maximal 20 mm) gewählt ist, daß eine Analyse der Zellen unter dem Mikroskop möglich ist.

Der Behälter ist zumindest im Bereich des Fensters aus einem Material mit einer für das Mikroskopieren hinreichenden Klarheit ausgeführt. Dieses ist z.B. Polystyrol oder Acrylglas. Diese Materialien weisen auch eine hinreichende Temperaturbeständigkeit auf. Grundsätzlich kommt auch eine Ausführung des optischen Fensters aus Glas in Betracht.

Das optische Fenster ist z.B. abdichtend mit dem Rand einer Öffnung des Behälters verbunden. Die Verbindung des optischen Fensters mit dem Behälter ist z.B. ausgeführt, wie eine der oben beschriebenen Verbindungen des Sterilfilters mit dem Behälter. Gemäß einer Ausgestaltung ist das Fenster integral mit dem Behälter ausgebildet, z.B. indem es aus einem Kunststoff besteht, der einteilig mit dem Kunststoff des übrigen Behälters verbunden ist. Der Behälter mit integralem optischen Fenster ist z.B. im Ein- oder Mehrkomponenten-Spritzgießverfahren aus einem oder mehreren Kunststoffen hergestellt.

Gemäß einer Ausgestaltung weist der Behälter an Boden und Kopf Einrichtungen zum Stapeln auf. Dies ermöglicht eine platzsparende Anordnung. Gemäß einer weiteren Ausgestaltung weist der Behälter an Boden und Kopf komplementäre Stützflächen und Führungsflächen zum Stapeln von mehreren Behältern mit identisch ausgebildeten Böden und Köpfen auf. An den Stützflächen ruhen die übereinandergestapelten Behälter aufeinander. Die Führungsflächen verhindern, daß die Behälter seitlich vom Stapel herunterfallen.

Gemäß einer Ausgestaltung besteht der Behälter ganz oder teilweise aus einem starren Material. Gemäß einer weiteren Ausgestaltung ist der Behälter ein im wesentlichen starrer Container.

Gemäß einer Ausgestaltung besteht der Behälter ganz oder teilweise aus einem flexiblen Material. Gemäß einer weiteren Ausgestaltung ist der Behälter ein Beutel. Ein als Porenmembran ausgebildetes Sterilfilter ist z.B. in einen Beutel integriert, in dem es eine Seitenwand des Beutels bildet, die randseitig mit mindestens einer weiteren Seitenwand des Beutels verbunden ist. Beispielsweise besteht der Beutel aus zwei übereinandergelegten Seitenwänden, die an den beiden Längsseiten und an einer Querseite miteinander verschweißt und an einer weiteren Querseite nicht miteinander verschweißt sind, so daß sie dort eine Öffnung bilden. Gemäß einem weiteren Beispiel bestehen sämtliche Seitenwände des Beutels aus dem Sterilfilter-Material. Gemäß einem weiteren Beispiel ist der Beutel ein Schlauchbeutel mit einer einzigen Seitenwand, die komplett oder abschnittsweise als Porenmembran ausgeführt ist. Die Öffnung eines Beutels kann einfach durch Zuknoten des Beutels an der Öffnung und/oder durch Zuschweißen des Beutels am Rand der Öffnung mittels einer geeigneten Schweißeinrichtung geschlossen werden. Insbesondere ein zuknotbarer Beutel ist z.B. in der Nähe der Öffnung oder insgesamt schlauchförmig.

Mischformen sind ebenfalls möglich, d.h. Behälter, die teilweise starr und teilweise flexibel sind, z.B. starre Behälter mit einem flexiblen Sterilfilter und/oder mit einer Öffnung in einem flexiblen (z.B. schlauchartigen) Teilbereich.

Gemäß einer Ausgestaltung weist der Behälter ein Beschriftungsfeld auf und/oder trägt eine Kennzeichnung. Dies ermöglicht dem Anwender eine Kennzeichnung der Zellkultur, die Verwechslungen mit eigenen oder fremden Zellkulturen verhindert.

Gemäß einer Ausgestaltung ist der Behälter ganz oder teilweise aus mindestens einem Kunststoff hergestellt. Bevorzugt wird der Behälter aus einem Kunststoff hergestellt, der autoklavierbar und temperierbar in einem für Zellkulturen geeigneten Bereich (z.B. 30°C bis 50°C) ist. Vorteilhaft ist auch die Verwendung von Kunststoff, der eine Betrachtung der Kulturen im Kulturgefäß mittels Mikroskop durch eine Wandung bzw. ein optisches Fenster des Behälters hindurch ermöglicht.

Gemäß einer Ausgestaltung des Verfahrens wird der Behälter vor dem Einfüllen der Zellen sterilisiert. Dies ist z.B. vor Wiederverwendung des Behälters zweckmäßig, kommt aber auch bei Behältern in Betracht, die sterilisiert vom Hersteller bereitgestellt werden, um Verunreinigungen ganz sicher auszuschließen.

Gemäß einer Ausgestaltung werden in das im Behälter enthaltene Kulturgefäß Zellen eingefüllt und danach der Behälter kontaminationsdicht verschlossen. Dies ist beispielsweise der Fall, wenn in den Behälter ein Kulturgefäß integriert ist. Es ist aber auch möglich, zunächst das Kulturgefäß in den Behälter einzusetzen und danach die Zellen in das im Behälter enthaltene Kulturgefäß einzufüllen.

Wenn das Kulturgefäß in den Behälter eingebracht werden muß, ist meist eine Ausgestaltung des Verfahrens zweckmäßig, bei der in das Kulturgefäß Zellen eingefüllt, das Kulturgefäß mit den eingefüllten Zellen in den Behälter eingebracht und danach der Behälter kontaminationsdicht verschlossen wird.

Gemäß einer Ausgestaltung werden außerhalb des Behälters Kulturbedingungen hergestellt und in den Behälter eingebracht. Dies ist beispielsweise der Fall, wenn ein mit Sterilfilter ausgestatteter Behälter in einen Brutschrank eingesetzt wird und wenn ein mit Anschlüssen ausgestatteter Behälter an eine externe Begasungseinrichtung angeschlossen wird. Dies ist auch dann der Fall, wenn der Behälter auf die Temperierplatte einer Temperiereinrichtung aufgesetzt wird.

Gemäß dem Verfahren wird ein Kulturbedingungen aufweisendes Gas sterilgefiltert und in den Behälter eingeleitet. Das Kulturbedingungen aufweisende Gas weist z.B. einen bestimmten Gehalt an CO₂, 02, Wasserdampf und gegebenenfalls eine bestimmte Temperatur auf.

Gemäß einer Ausgestaltung wird durch Wärmeübertragung zwischen dem Behälter und einer externen Temperiereinrichtung eine bestimmte Temperatur im Behälter hergestellt.

Gemäß einer Ausgestaltung werden den Zellen unter Kulturbedingungen mindestens ein Medium zugeführt und/oder mindestens eine Probe entnommen.

Gemäß einer Ausgestaltung werden die Zellen im Behälter unter Kulturbedingungen beobachtet. Die Beobachtung erfolgt bevorzugt durch ein optisches Fenster mit Hilfe eines Mikroskopes.

Gemäß einer Ausgestaltung wird im Behälter durch Verdampfen einer Flüssigkeit eine das Verdampfen von Kulturmedium verhindernde Atmosphäre eingestellt. Bei dieser Ausgestaltung werden Kulturbedingungen direkt im Inneren des Behälters hergestellt. Die Flüssigkeit ist z.B. steriles Wasser.

Gemäß einer Ausgestaltung wird das Kulturgefäß aus dem Behälter entnommen, die Zellkultur einer Kontrolle unterzogen und danach das Kulturgefäß in denselben oder in einen frischen Behälter eingesetzt, der Behälter kontaminationsdicht verschlossen und werden Kulturbedingungen für die Zellen im Behälter hergestellt.

Gemäß einer Verwechslungen vermeidenden Ausgestaltung wird der Behälter beschriftet und/oder mit einer Kennzeichnung versehen.

Schließlich wird gemäß einer Ausgestaltung der Behälter nach Beendigung der Inkubation weggeworfen, d.h. in den Abfall gegeben. Dies erleichtert die Handhabung. Ein für Wiederverwendung erforderliches Sterilisieren des Behälters entfällt.

Die Erfindung wird nachfolgend anhand der anliegenden Zeichnungen erläutert, die grobschematisch verschiedene Ausführungsbeispiele zeigen.

Bei der nachfolgenden Beschreibung der verschiedenen Ausführungsbeispiele sind dieselbe Benennung aufweisende Elemente der Vorrichtung mit denselben Bezugsziffern bezeichnet, wobei unterschiedliche Ausführungen durch einen nachgestellten Punkt und eine nachgestellte, individuelle Ziffer versehen sind.

Ein im wesentlichen kastenförmiger Behälter 1.1. hat ein integriertes Kulturgefäß 2.1, das mit einem Boden 3.1 des Behälters 1.1 fest verbunden ist. Der flache Boden 3.1 ist auf eine Temperierplatte 4 einer Temperiereinrichtung 5 aufsetzbar.

In einer Deckwand 6.1 hat der Behälter 1.1 eine verschließbare Öffnung 7.1 zum Einbringen und Entnehmen von Zellen, Kulturmedium usw.. Die Öffnung 7.1 ist verhältnismäßig klein ausgebildet, da nur die vorerwähnten Medien hindurchgeführt werden müssen. Gegebenenfalls wird vor dem Verschließen der Öffnung 7.1 durch diese eine Zellkulturbedingungen herstellende Atmosphäre in den Behälter 1.1 eingeleitet. Dies kann auch wiederholt werden.

Ferner ist ein Behälter 1.2 gezeigt, der in der Deckwand 6.2 eine größere verschließbare Öffnung 7.2 aufweist, durch die ein Kulturgefäß 2.2 mit einem Deckel 8 einsetzbar und entnehmbar ist. Außerdem sind die Abmessungen des Behälters 1.2 auf die Größe des einsetzbaren Kulturgefäßes 2.2 mit Deckel 8 abgestimmt. Der flache Boden 3.1 ist wiederum auf die Temperierplatte 4 einer Temperiereinrichtung 5 aufsetzbar.

Der Behälter 1.3 unterscheidet sich von dem vorbeschriebenen dadurch, daß er so bemessen ist, daß mehrere Kulturgefäße 2.2 mit Deckel 8 übereinandergestapelt in ihn hineinpassen. Außerdem ist die Öffnung 7.3 in der Deckwand 6.3 größer ausgeführt, so daß mehrere Kulturgefäße 2.2 mit Deckel 8 übereinandergestapelt hindurch passen. Gegebenenfalls wird in den Behälter 1.3 durch die Öffnung 7.3 vor dem Verschließen der Öffnung 7.3 eine Zellkulturbedingungen herstellende Atmosphäre eingeleitet. Dies kann auch wiederholt werden.

Der Behälter 1.4 weist im Unterschied zum Behälter 1.2 in einer Wandung 7.1 ein Sterilfilter 9 auf. Ferner hat er ein in die Deckwand 6.4 integriertes Sichtfenster 10 zum Beobachten der Zellen im Kulturgefäß 2.2 mittels eines Mikroskops. Zusätzlich oder statt des Sichtfensters 10 ist in einen Boden 3.1 des Behälters 1.4 ein Sichtfenster 10.1 integriert, das ein Beobachten der Zellen im Kulturgefäß 2.2 mittels eines inversen Mikroskopes ermöglicht.

Der Behälter 1.4 ist zusammen mit herkömmlichen Zellkulturgefäßen 2.2 in einem Brutschrank 11 inkubierbar. Die in dem Brutschrank 11 durch einen Gasanschluß 12 eingespeiste Atmosphäre dringt durch das Sterilfilter 9 in das Innere des Behälters 1.4 ein und bis zu den Zellen im Kulturgefäß 2.2 vor.

Der Behälter 1.5 weist im Unterschied zum Behälter 1.4 in einer Wandung 7.2 einen Anschluß 13 mit einem Durchgang zum Inneren des Behälters 1.5 und einem in dem Anschluß integrierten Ventil 14 zum Öffnen und Sperren des Durchganges auf. Der Anschluß 13 ist an eine externe Begasungseinrichtung anschließbar.

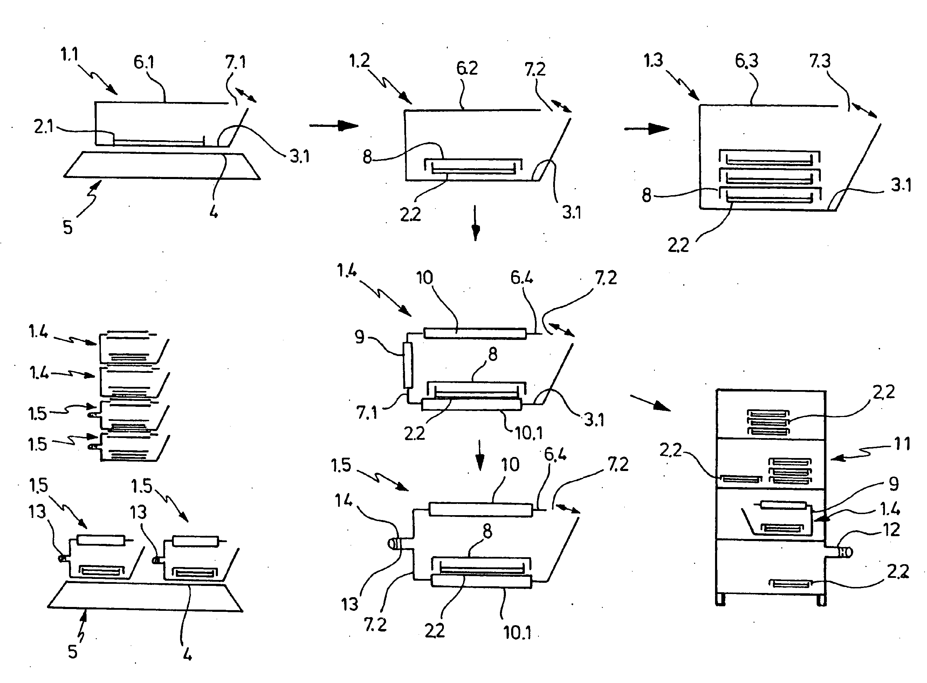

Mehrere Behälter 1.5 sind z.B. auf der Temperierplatte 4 einer Temperiereinrichtung 5 plazierbar, wobei gleichzeitig eine Begasung über die Anschlüsse 13 mittels einer nicht gezeigten externen Begasungseinrichtung erfolgt. Auch dies ist in der Zeichnung dargestellt.

Ferner zeigt die Zeichnung die freie Kombinierbarkeit von Behältern 1.4 und Behältern 1.5, die übereinandergestapelt gemeinsam in einen Brutschrank 11 einstellbar sind, wobei die Behälter 1.5 an eine externe Begasungseinrichtung anschließbar sind, um darin eine spezielle Atmosphäre herzustellen.

## Patentansprüche

1. Vorrichtung zum Inkubieren von Zellen mit einem sterilen oder sterilisierbaren, tragbaren Behälter (1) zum kontaminationsdichten Umschließen mindestens eines integrierten und/oder einsetzbaren Kulturgefäßes (2) zur Aufnahme von Zellen mit mindestens einer verschließbaren Öffnung (7) zum Einbringen und/oder Entnehmen von Zellen und/oder Kulturmedium und/oder eines Kulturgefäßes (2) in den und/oder aus dem Behälter (1) und mindestens einer Einrichtung zum Herstellen von Kulturbedingungen (11) in dem Behälter, die mindestens einen Sterilfilter (9) in mindestens einer Wandung (7.1) des Behälters (1.4) umfaßt.

2. Vorrichtung nach Anspruch 1 mit mindestens einem Kulturgefäß (2.1, 2.2).

3. Vorrichtung nach Anspruch 2 mit mindestens einem in dem Behälter (1.1) integrierten Kulturgefäß (2.1).

4. Vorrichtung nach Anspruch 3 mit mindestens einem fest und untrennbar mit dem Behälter (1.1) verbundenen Kulturgefäß (2.1).

5. Vorrichtung nach einem der Ansprüche 2 bis 4 mit Einrichtungen zum lösbaren Verbinden mindestens eines Kulturgefäßes (2) mit dem Behälter (1).

6. Vorrichtung nach einem der Ansprüche 1 bis 5 mit einem für das Umschließen und/oder Einbringen und/oder Entnehmen mindestens einer Multiwell-Kulturplatte geeigneten Behälter (1).

7. Vorrichtung nach einem der Ansprüche 1 bis 6 mit einem für das Umschließen und/oder Einbringen und/oder Entnehmen mindestens einer Kulturflasche geeigneten Behälter (1).

8. Vorrichtung nach einem der Ansprüche 1 bis 7 mit einem für das Umschließen und/oder Einbringen und/oder Entnehmen mindestens einer Kulturschale geeigneten Behälter (1).

9. Vorrichtung nach einem der Ansprüche 1 bis 8, bei der die Einrichtung zum Herstellen von Kulturbedingungen einen Brutschrank (11) mit integrierter Temperiereinrichtung und Begasungseinrichtung zur Aufnahme mindestens eines Behälters (1.4) umfaßt.

10. Vorrichtung nach einem der Ansprüche 1 bis 9, bei der die Einrichtung zum Herstellen von Kulturbedingungen mindestens einen Anschluß (14) des Behälters (1.5) mit einem eine Wandung des Behälters querenden Durchgang zum Anschließen des Behälters (1.5) an mindestens eine Begasungseinrichtung umfaßt.

11. Vorrichtung nach Anspruch 10, bei der die Einrichtung zum Herstellen von Kulturbedingungen eine Begasungseinrichtung und mindestens einen Schlauch umfaßt, über den die Begasungseinrichtung mit mindestens einem Anschluß (13) verbunden oder verbindbar ist.

12. Vorrichtung nach Anspruch 10 oder 11, bei der der Anschluß (13) ein Ventil (14) zum Öffnen und Schließen des Durchgangs umfaßt.

13. Vorrichtung nach einem der Ansprüche 1 bis 12, bei der der Behälter (1.1) einen ebenen Boden (3.1) zum Aufsetzen auf eine Temperierplatte (4) einer Temperiereinrichtung (5) aufweist.

14. Vorrichtung nach einem der Ansprüche 1 bis 13, bei der die Einrichtung zum Herstellen von Kulturbedingungen mindestens ein im Behälter (1) integrierte und/oder in den Behälter (1) einbringbares offenes Gefäß zum Verdampfen einer in das Gefäß eingegebenen Flüssigkeit umfaßt.

15. Vorrichtung nach einem der Ansprüche 1 bis 14, bei der die Einrichtung zum Herstellen von Kulturbedingungen eine Mischeinrichtung umfaßt, auf der der Behälter angeordnet oder anordenbar ist, um den Behälter (1) einer Mischbewegung zu unterziehen.

16. Vorrichtung nach einem der Ansprüche 1 bis 15, bei der mindestens eine Öffnung (7) des Behälters (1) wiederverschließbar ist.

17. Vorrichtung nach einem der Ansprüche 1 bis 16, bei der der Behälter (1.4) mindestens ein optisches Fenster (10) zum Betrachten der Zellen im Kulturgefäß (2.2) aufweist.

18. Vorrichtung nach einem der Ansprüche 1 bis 17, bei der der Behälter (1) an Boden und Kopf Einrichtungen zum Stapeln aufweist.

19. Vorrichtung nach Anspruch 18, bei der der Behälter (1) an Boden und Kopf komplementäre Stützflächen und Führungsflächen zum Stapeln von mehreren Behältern (1) mit identisch ausgebildeten Böden und Köpfen aufweist.

20. Vorrichtung nach einem der Ansprüche 1 bis 19, bei der der Behälter (1) ganz oder teilweise aus einem starren Material besteht.

21. Vorrichtung nach Anspruch 20, bei der der Behälter (1) ein im wesentlichen starrer Container ist.

22. Vorrichtung nach einem der Ansprüche 1 bis 21, bei der der Behälter (1) ganz oder teilweise aus einem flexiblen Material besteht.

23. Vorrichtung nach Anspruch 22, bei der der Behälter (1) ein Beutel ist.

24. Vorrichtung nach einem der Ansprüche 1 bis 23, bei der der Behälter (1) ein Beschriftungsfeld aufweist und/oder eine Kennzeichnung trägt.

25. Vorrichtung nach einem der Ansprüche 1 bis 24, bei der der Behälter (1) ganz oder teilweise aus mindestens einem Kunststoff hergestellt ist.

26. Vorrichtung nach einem der Ansprüche 1 bis 25, bei der der Behälter (1) und/oder das Zellkulturgefäß (2) ein Einmalteil ist/sind.

27. Verfahren zum Inkubieren von Zellenunter Verwendung einer Vorrichtung gemäß einem der Ansprüche 1 bis 26, bei dem mindestens ein Zellen enthaltendes Kulturgefäß (2) von einem sterilen, tragbaren Behälter (1) kontaminationsdicht umschlossen bereitgestellt wird und Kulturbedingungen für die Zellen im Behälter (1) hergestellt werden, bei dem ein Kulturbedingungen aufweisendes Gas sterilgefiltert und in den Behälter (1) eingeleitet wird.

28. Verfahren nach Anspruch 27, bei dem der Behälter (1) vor dem Einfüllen der Zellen sterilisiert wird.

29. Verfahren nach Anspruch 27 oder 28, bei dem in das im Behälter (1) enthaltene Kulturgefäß (2) Zellen eingefüllt und danach der Behälter (1) kontaminationsdicht verschlossen wird.

30. Verfahren nach Anspruch 29, bei dem in das Kulturgefäß (2) Zellen eingefüllt, das Kulturgefäß (2) mit den eingefüllten Zellen in den Behälter (1) eingebracht und danach der Behälter (1) kontaminationsdicht verschlossen wird.

31. Verfahren nach einem der Ansprüche 27 bis 30, bei dem außerhalb des Behälters (1) Kulturbedingungen hergestellt und in den Behälter (1) eingebracht werden.

32. Verfahren nach einem der Ansprüche 27 bis 31, bei dem durch Wärmeübertragung zwischen Behälter (1) und einer externen Temperiereinrichtung (5) eine bestimmte Temperatur im Behälter (1) hergestellt wird.

33. Verfahren nach einem der Ansprüche 27 bis 32, bei dem den Zellen unter Kulturbedingungen mindestens ein Medium zugeführt und/oder mindestens eine Probe entnommen wird.

34. Verfahren nach einem der Ansprüche 27 bis 33, bei dem die Zellen im Behälter (1) unter Kulturbedingungen beobachtet werden.

35. Verfahren nach einem der Ansprüche 27 bis 34, bei dem im Behälter (1) durch Verdampfen einer Flüssigkeit eine das Verdampfen von Kulturmedium verhindernde Atmosphäre eingestellt wird.

36. Verfahren nach einem der Ansprüche 27 bis 35, bei dem das Kulturgefäß (2) aus dem Behälter (1) entnommen, die Zellkultur einer Kontrolle unterzogen und danach das Kulturgefäß (2) in denselben oder in einen frischen Behälter (1) eingesetzt, der Behälter (1) kontaminationsdicht verschlossen und Kulturbedingungen für die Zellen im Behälter (1) hergestellt werden.

37. Verfahren nach einem der Ansprüche 27 bis 36, bei dem der Behälter (1) beschriftet und/oder mit einer Kennzeichnung versehen wird.

38. Verfahren nach einem der Ansprüche 27 bis 37, bei dem der Behälter (1) nach Beendigung der Inkubation weggeworfen wird.

## Claims

1. Device for the incubation of cells comprising a sterile or sterilisable, portable receptacle (1) for enclosing in a contamination-proof manner at least one integrated and/or insertable culture vessel (2) for accommodating cells with at least one closable opening (7) for introducing and/or removing cells and/or culture medium and/or a culture vessel (2) into and/or from the receptacle (1) and at least one device for creating culture conditions (11) in the receptacle, which comprises at least one sterile filter (9) in at least one wall (7.1) of the receptacle (1.4).

2. Device according to Claim 1 comprising at least one culture vessel (2.1, 2.2).

3. Device according to Claim 2 comprising a culture vessel (2.1) integrated in the receptacle (1.1).

4. Device according to Claim 3, comprising at least one culture vessel (2.1) connected fixedly and inseparably to the receptacle (1.1).

5. Device according to one of Claims 2 to 4 comprising devices for the releasable connection of at least one culture vessel (2) to the receptacle (1).

6. Device according to one of Claims 1 to 5, comprising a receptacle (1) suitable for enclosing and/or inserting and/or removing at least one multiwell culture plate.

7. Device according to one of Claims 1 to 6, comprising a receptacle (1) suitable for enclosing and/or inserting and/or removing at least one culture flask.

8. Device according to one of Claims 1 to 7 comprising a receptacle (1) suitable for enclosing and/or inserting and/or removing at least one culture dish.

9. Device according to one of Claims 1 to 8, in which the device for creating culture conditions comprises an incubator (11) with integral tempering device and gassing device for accommodating at least one receptacle (1.4).

10. Device according to one of Claims 1 to 9, in which the device for creating culture conditions comprises at least one connection (14) of the receptacle (1.5) with a through-passage traversing a wall of the receptacle for attaching the receptacle (1.5) to at least one gassing device.

11. Device according to Claim 10, in which the device for creating culture conditions comprises a gassing device and at least one tube via which the gassing device is connected or may be connected to at least one connection (13).

12. Device according to Claim 10 or 11, in which the connection (13) comprises a valve (14) for opening and closing the through-passage.

13. Device according to one of Claims 1 to 12, in which the receptacle (1.1) comprises a planar base (3.1) for positioning on a tempering plate (4) of a tempering device (5).

14. Device according to one of Claims 1 to 13, in which the device for creating culture conditions comprises at least one open vessel which is integrated in the receptacle (1) and/or which may be inserted into the receptacle (1) for evaporating a liquid introduced into the vessel.

15. Device according to one of Claims 1 to 14, in which the device for creating culture conditions comprises a mixing device on which the receptacle is arranged or may be arranged in order to subject the receptacle (1) to a mixing motion.

16. Device according to one of Claims 1 to 15, in which at least one opening (7) of the receptacle (1) is reclosable.

17. Device according to one of Claims 1 to 16, in which the receptacle (1.4) comprises at least one optical window (10) for observing the cells in the culture vessel (2.2).

18. Device according to one of Claims 1 to 17, in which the receptacle (1) comprises devices for stacking at the bottom and top.

19. Device according to Claim 18, in which the receptacle (1) comprises complementary support surfaces and guide surfaces at the bottom and top for stacking a plurality of receptacles (1) with identically configured bases and tops.

20. Device according to one of Claims 1 to 19, in which the receptacle (1) consists entirely or partially of a rigid material.

21. Device according to Claim 20, in which the receptacle (1) is a substantially rigid container.

22. Device according to one of Claims 1 to 21, in which the receptacle (1) consists entirely or partially of a flexible material.

23. Device according to Claim 22, in which the receptacle (1) is a bag.

24. Device according to one of Claims 1 to 23, in which the receptacle (1) comprises a label and/or carries a marker.

25. Device according to one of Claims 1 to 24, in which the receptacle (1) is produced entirely or partially from at least one plastic.

26. Device according to one of Claims 1 to 25, in which the receptacle (1) and/or the cell culture vessel (2) is/are a single-use item.

27. Method for the incubation of cells by using a device according to one of Claims 1 to 26, in which at least one culture vessel (2) containing cells is provided enclosed by a sterile, portable receptacle (1) in a contamination-proof manner and culture conditions are created for the cells in the receptacle (1), in which a gas comprising culture conditions is filtered in a sterile manner and introduced into the receptacle (1).

28. Method according to Claim 27, in which the receptacle (1) is sterilised before filling with cells.

29. Method according to Claim 27 or 28, in which cells are filled into the culture vessel (2) contained in the receptacle (1) and subsequently the receptacle (1) is closed in a contamination-proof manner.

30. Method according to Claim 29, in which cells are filled into the culture vessel (2), the culture vessel (2) is inserted with the introduced cells into the receptacle (1) and subsequently the receptacle (1) is closed in a contamination-proof manner.

31. Method according to one of Claims 27 to 30, in which culture conditions are created outside the receptacle (1) and introduced into the receptacle (1).

32. Method according to one of Claims 27 to 31, in which as a result of heat transmission between the receptacle (1) and an external tempering device (5) a specific temperature is generated in the receptacle (1).

33. Method according to one of Claims 27 to 32, in which at least one medium is supplied to the cells under culture conditions and/or at least one sample is removed.

34. Method according to one of Claims 27 to 33, in which the cells are observed in the receptacle (1) under culture conditions.

35. Method according to one of Claims 27 to 34, in which an atmosphere preventing the evaporation of culture medium is established in the receptacle (1) by evaporating a liquid.

36. Method according to one of Claims 27 to 35, in which the culture vessel (2) is removed from the receptacle (1), the cell culture subjected to a control and subsequently the culture vessel (2) is inserted into the same receptacle or into a new receptacle (1), the receptacle (1) is closed in a contamination-proof manner and culture conditions are created for the cells in the receptacle (1).

37. Method according to one of Claims 27 to 36, in which the receptacle (1) is labelled and/or provided with a marker.

38. Method according to one of Claims 27 to 37, in which the receptacle (1) is discarded after the incubation is completed.

## Revendications

1. Dispositif pour l'incubation de cellules, avec un récipient (1) portable stérile ou stérilisable pour enfermer de façon étanche contre la contamination au moins un flacon de culture (2) intégré eUou insertable pour recevoir des cellules, avec au moins une ouverture (7) verrouillable pour charger et/ou prélever des cellules et/ou du milieu de culture et/ou d'un flacon de culture (2) dans le et/ou du récipient (1), et avec au moins une installation (11) pour établir des conditions de culture dans le récipient, qui comporte au moins un filtre de stérilisation (9) dans au moins une paroi (7.1) du récipient (1.4).

2. Dispositif selon la revendication 1, avec au moins un flacon de culture (2.1, 2.2).

3. Dispositif selon la revendication 2, avec au moins un flacon de culture (2.1) qui est intégré dans le récipient (1.1).

4. Dispositif selon la revendication 3, avec au moins un flacon de culture (2.1) qui est relié au récipient (1.1) de façon ferme et inséparable.

5. Dispositif selon l'une quelconque des revendications 2 à 4, avec des installations pour la connexion amovible d'au moins un flacon de culture (2) avec le récipient (1).

6. Dispositif selon l'une quelconque des revendications 1 à 5, avec un récipient (1) qui est susceptible d'enfermer et/ou charger et/ou prélever au moins une plaque de culture du type multipuits.

7. Dispositif selon l'une quelconque des revendications 1 à 6, avec un récipient (1) qui est susceptible d'enfermer et/ou charger et/ou prélever au moins une bouteille de culture.

8. Dispositif selon l'une quelconque des revendications 1 à 7, avec un récipient (1) qui est susceptible d'enfermer et/ou charger et/ou prélever au moins une boîte de culture.

9. Dispositif selon l'une quelconque des revendications 1 à 8, dans lequel l'installation pour établir des conditions de culture comporte un incubateur (11) avec des installations pour régler la température et pour mettre sous gaz intégrées, qui est adapté à recevoir au moins un récipient (1.4).

10. Dispositif selon l'une quelconque des revendications 1 à 9, dans lequel l'installation pour établir des conditions de culture comporte au moins un accouplement (14) du récipient (1.5) avec un passage traversant la paroi du récipient pour accoupler le récipient (1.5) avec au moins une installation pour mettre sous gaz.

11. Dispositif selon la revendication 11, dans lequel l'installation pour établir des conditions de culture comporte une installation pour mettre sous gaz et au moins un tuyau à travers lequel l'installation pour mettre sous gaz est reliée ou adaptée à être reliée avec au moins un accouplement (13).

12. Dispositif selon la revendication 10 ou 11, dans lequel l'accouplement (13) comporte une vanne (14) pour ouvrir ou fermer le passage.

13. Dispositif selon l'une quelconque des revendications 1 à 12, dans lequel le récipient (1.1) comporte un fond plat (3.1) pour être mis sur une plaque à régulation de température (4) d'une installation pour régler la température (5).

14. Dispositif selon l'une quelconque des revendications 1 à 13, dans lequel l'installation pour établir des conditions de culture comporte au moins un flacon ouvert intégré dans le récipient (1) ou adapté à être inséré dans le récipient (1) pour évaporer un liquide qui était alimenté dans le flacon.

15. Dispositif selon l'une quelconque des revendications 1 à 14, dans lequel l'installation pour établir des conditions de culture comporte une installation à mélanger sur laquelle le récipient est arrangé ou adapté à être arrangé, afin de soumettre le récipient (1) à une agitation de mélange.

16. Dispositif selon l'une quelconque des revendications 1 à 15, dans lequel au moins une ouverture (7) du récipient (1) est refermable.

17. Dispositif selon l'une quelconque des revendications 1 à 16, dans lequel le récipient (1.4) a au moins une fenêtre optique (10) pour observer les cellules dans le flacon de culture (2.2).

18. Dispositif selon l'une quelconque des revendications 1 à 17, dans lequel le récipient (1) a des installations pour entasser dans le fond et sur la tête.

19. Dispositif selon la revendication 18, dans lequel le récipient (1) a au fond et sur la tête des surfaces de support et des surfaces de guidage complémentaires pour entasser plusieurs récipients (1) avec des fond et têtes qui sont formés identiques.

20. Dispositif selon l'une quelconque des revendications 1 à 19, dans lequel le récipient (1) consiste entièrement ou partiellement d'un matériel rigide.

21. Dispositif selon la revendication 20, dans lequel le récipient (1) est un conteneur essentiellement rigide.

22. Dispositif selon l'une quelconque des revendications 1 à 21, dans lequel le récipient (1) consiste entièrement ou partiellement d'un matériel flexible.

23. Dispositif selon la revendication 23, dans lequel le récipient (1) est un sachet.

24. Dispositif selon l'une quelconque des revendications 1 à 23, dans lequel le récipient comporte un champ d'inscription et/ou une caractérisation.

25. Dispositif selon l'une quelconque des revendications 1 à 24, dans lequel le récipient (1) est fait entièrement ou partiellement d'au moins une matière plastique.

26. Dispositif selon l'une quelconque des revendications 1 à 25, dans lequel le récipient (1) et/ou le flacon de culture de cellules (2) est une pièce à utilisation unique.

27. Méthode pour l'incubation de cellules en utilisant un dispositif selon l'une quelconque des revendications 1 à 26, dans laquelle au moins un flacon de culture (2) contenant des cellules est fourni étant enfermé de façon étanche contre la contamination par un récipient (1) portable et stérile et des conditions de culture sont établies pour les cellules dans le récipient (1), et dans laquelle un gaz ayant des conditions de culture est filtré en un état stérile et chargé dans le récipient (1).

28. Méthode selon la revendication 27, dans laquelle le récipient (1) est stérilisé avant le remplissage avec les cellules.

29. Méthode selon la revendication 27 ou 28, dans laquelle des cellules sont chargées dans le flacon de culture (2) contenu dans le récipient (1) et après, le récipient (1) est fermé de façon étanche contre la contamination.

30. Méthode selon la revendication 29, dans laquelle des cellules sont chargées dans le flacon de culture (2), le flacon de culture (2) avec les cellules chargées est mis dans le récipient (1) et après, le récipient (1) est fermé de façon étanche contre la contamination.

31. Méthode selon l'une quelconque des revendications 27 à 30, dans laquelle des conditions de culture sont établis au dehors du récipient (1) et sont chargées dans le récipient (1).

32. Méthode selon l'une quelconque des revendications 27 à 31, dans laquelle une certaine température est établie dans le récipient (1) par transfert de chaleur entre le récipient (1) et une installation de réglage de température externe (5).

33. Méthode selon l'une quelconque des revendications 27 à 32, dans laquelle au moins un milieu est fourni aux cellules et/ou au moins un échantillon est enlevée sous des conditions de culture.

34. Méthode selon l'une quelconque des revendications 27 à 33, dans laquelle les cellules dans le récipient (1) sont observées sous des conditions de culture.

35. Méthode selon l'une quelconque des revendications 27 à 34, dans laquelle une atmosphère empêchant l'évaporation du milieu de culture est établie dans le récipient (1) en évaporant un liquide.

36. Méthode selon l'une quelconque des revendications 27 à 35, dans laquelle le flacon de culture (2) est enlevé du récipient (1), la culture de cellules est soumise à un contrôle et après, le flacon de culture est mis dans le même ou dans un nouveau récipient (1), le récipient (1) est fermé de façon étanche contre la contamination et des conditions de culture sont établies pour les cellules dans le récipient (1).

37. Méthode selon l'une quelconque des revendications 27 à 36, dans laquelle le récipient (1) est étiqueté et/ou pourvu d'une marque.

38. Méthode selon l'une quelconque des revendications 27 à 37, dans laquelle le récipient (1) est mis au rebut après la complétion de l'incubation.
